# EUROPEAN PATENT APPLICATION

(11) **EP 4 625 426 A1**
(43) Date of publication of application: **01.10.2025**
(21) Application number: 24167852.3
(22) Date of filing: 29.03.2024
(51) Int. Cl.: G16H 10/60, G06F 40/186, G16H 15/00

(54) **TEMPLATE DETECTION AND EXTRACTION OF MEDICAL DATA**

(71) Applicant: Koninklijke Philips N.V., 5656 AG Eindhoven (NL)
(72) Inventor: BASTIANELLI, Emanuele, Eindhoven (NL); HARMA, Aki Sakari, Eindhoven (NL); ZEGELAAR, Eva, 5656AG Eindhoven (NL); PEREVERZEVA, Anna, Eindhoven (NL)
(74) Representative: Philips Intellectual Property & Standards

(57) **Abstract**

Techniques for extracting medical data from medical records based on templates are disclosed. A medical record comprising medical data is accessed. A trained model classifies the medical record to a template, the template comprising a layout of the medical record, a key-value pair, and/or a data structure in the medical record. A language model is selected to extract the medical data. The medical data is extracted from the medical record based on the detected template and using the language model. Output data is generated based on a predetermined using the extracted medical data.

## Description

### FIELD OF THE INVENTION

This application relates to template detection and extraction of medical data. Various embodiments can train and apply models to detect templates for medical records and extract medical data based on the identified templates.

### BACKGROUND OF THE INVENTION

Medical records are used for systematic documentation of medical history and care of subjects (e.g., patients) across time. A medical record can include a variety of types of "notes" entered over time by healthcare professionals to record, for example, observations, administration of drugs and therapies, orders for the administration of drugs and therapies, test results, medical imaging results, reports, or the like. Maintenance of complete and accurate medical records is a requirement of healthcare providers and may be enforced through licensing or certification processes. As used herein, a medical record can refer to written (e.g., paper notes), physical (e.g., image films), or digital records for individual subjects and for the body of information found therein. Medical records are typically compiled and maintained by healthcare providers. Medical records can also include personal health records (PHR) that are maintained by subjects themselves (e.g., on third-party websites).

Medical records can include electronic health records (EHRs), which can be systematized collections of subject or population health information in a digital format. EHRs can be shared across different healthcare settings (e.g., facilities, providers), such as shared through network-connected, enterprise-wide information systems or other information networks and exchanges. EHRs may include various data, such as demographics, medical history, medication and allergies, immunization status, laboratory test results, radiology images, vital signs, personal statistics (e.g., age, weight), and billing information.

### SUMMARY OF THE INVENTION

Apparatuses, systems, and methods for medical data extraction are disclosed. The disclosed technology can be used to detect a template according to which a received medical record was prepared and extract medical data from the medical record based on the detected template. For example, the disclosed technology can be used to define and/or detect templates for structured, unstructured, and/or or semi-structured medical records (e.g., paper, physical, or digital records) and extract data from the medical records based on the templates.

In accordance with at least one example disclosed herein, a computer-implemented method is disclosed for automatically templatizing medical records data. A medical record comprising medical data is accessed. The medical record is classified to an appropriate template using a trained model, the template comprising a layout, a key-value pair, or a data structure in the medical record. Classifying the medical record can include generating a confidence score indicating a confidence that the medical record is associated with the template. In some implementations, the medical record is discarded when the confidence score does not exceed a threshold. In some implementations, classifying the medical record includes determining a set of template descriptors associated with the medical record, and extracting the medical data includes identifying at least one extractor to extract the medical data based on the set of template descriptors. A language model is selected based on the classified template, and the medical data is extracted from the medical record by applying the selected language model to the accessed medical record in accordance with the classified template. Output data according to a predetermined standard is generated using the extracted medical data. For example, the output data can be generated based on a Fast Healthcare Interoperability Resources (FHIR) standard. In some implementations, the generated output data is compared to known data of a subject associated with the medical record. In these and other implementations, the output data is discarded when the output data does not match the known data beyond a threshold accuracy.

In various embodiments, the trained model includes a classifier model. To train the classifier model, a plurality of medical records is received comprising a plurality of respective medical data, the plurality of medical records having respective templates. A training dataset is generated using the plurality of medical records. Generating the training dataset can include identifying at least one data structure in the plurality of medical records. The classifier model is trained using the generated training dataset to detect templates of received medical records to perform medical data extraction.

In various embodiments, the classifier model is retrained. The trained classifier model is evaluated using a testing dataset to determine an accuracy of the trained classifier model, the testing dataset comprising a portion of the generated training dataset. The trained classifier model is retrained when the accuracy of the trained classifier model does not exceed a threshold accuracy. Retraining the classifier model includes adjusting a weight associated with the classifier model or training the classifier model at least a second time.

In various embodiments, training the classifier model includes generating a template descriptor repository comprising a plurality of template types and template descriptors for the template types, wherein the template descriptors include section extractors associated with the template types, the section extractors comprising a parser, a natural language processing (NLP) model, or both.

In accordance with other examples disclosed herein, a computing system is disclosed comprising at least one processor and at least one memory carrying instructions configured to cause the computing system to perform one or more methods disclosed herein.

In accordance with other examples, a non-transitory computer-readable medium is disclosed carrying instructions that, when executed by a processor or a computing system, cause performance of one or more methods disclosed herein.

### BRIEF DESCRIPTION OF THE DRAWINGS

Fig. 1 is a block diagram illustrating a computing device implementing a system for medical data extraction arranged in accordance with principles of the present disclosure.
Fig. 2 is a block diagram illustrating a workflow for medical data extraction in accordance with principles of the present disclosure.
Fig. 3 is a block diagram illustrating a workflow for template discovery and extraction in accordance with principles of the present disclosure.
Fig. 4 is a block diagram illustrating a workflow for template detection in accordance with principles of the present disclosure.
Fig. 5 is a block diagram illustrating a workflow for garbage detection in accordance with principles of the present disclosure.
Fig. 6 is a sequence diagram illustrating a process for medical data extraction in accordance with principles of the present disclosure.

### DETAILED DESCRIPTION OF EMBODIMENTS

The following description of certain examples is merely illustrative in nature and is in no way intended to limit the disclosed technology or its applications or uses. In the following detailed description of examples of the present apparatuses, systems, and methods, reference is made to the accompanying drawings which form a part hereof, and in which are shown by way of illustration specific examples in which the described apparatuses, systems and methods can be practiced. These examples are described in sufficient detail to enable those skilled in the art to practice the presently disclosed apparatuses, systems, and methods, and it is to be understood that other examples may be utilized and that structural and logical changes may be made without departing from the spirit and scope of the present disclosure. Moreover, for the purpose of clarity, detailed descriptions of certain features will not be discussed when they would be apparent to those with skill in the art so as not to obscure the description of the present system. The following detailed description is therefore not to be taken in a limiting sense, and the scope of the present technology is defined only by the appended claims.

Healthcare providers face difficult technical challenges related to extracting medical data contained in medical records. Medical records are often prepared according to templates, which may include various parts, such as tables, key-value pairs, typed or written text (e.g., notes), and so forth. But these templates may not be standardized or uniform (e.g., across providers, facilities, systems). Accordingly, medical records may use varied and complicated template formats, which may make it difficult to accurately extract medical data (e.g., using automated processes or tools). For example, when a document with an unknown format is accessed or received by an automatic extraction process, the automatic extraction process may be unable to make sense of medical data in the document, which may lead to errors or incomplete data extraction. For example, these issues may occur when a subject's medical records are transferred from one medical facility to another medical facility that uses a different format. This may cause the subject's care providers to miss important information about the subject's medical history (e.g., conditions, medications) and lead to substandard care (e.g., fail to order tests for monitoring conditions, prescribe medications that interact). Therefore, it would be advantageous to be able to learn or detect templates or template types for medical records, such that medical data can be extracted from the medical records with improved accuracy and efficiency.

Disclosed herein are systems and related methods for extracting medical data from medical records. Various embodiments classify medical records (e.g., using one or more trained models) according to respective templates and extract medical data based on the classified templates. The disclosed technology can provide (e.g., generate and/or train) one or more models to learn different medical record templates and use these templates to detect templates for received medical records (e.g., newly-generated or received medical records). Detected templates are used for extracting medical data from the received medical records. In various embodiments, the extracted medical data can be transformed or reformatted based on one or more standards, such as the Fast Healthcare Interoperability Resources (FHIR) standard. In various embodiments, the disclosed technology selects and uses an appropriate language model to extract medical data from medical records based on detected templates. As used herein, a language model can refer to a natural language processing (NLP) model or other tool configured to process language inputs in various formats, such as text and/or audio inputs. For example, a language model can comprise a NLP extraction process used to extract data from language inputs, which may rely on an underlying probability distribution over a sequence of words in some embodiments. In some implementations, a language model can comprise a trained model, such as a named-entity recognition process, which may rely on or include a trained model. In these and other embodiments, a language model may not comprise or use a trained model, such as simple models for extracting data based on key-value pairs.

Medical records can be composed using dashboard tools that allow a clinician to fill in a predefined document template whose key fields are typed or filled with terms chosen from predefined drop-down menus. In other examples, medical records can be prepared using paper forms with predefined parts that are filled in (e.g., by a provider). In still other examples, medical records can be generated completely or substantially in an automatic way using computing systems (e.g., imaging devices, personal computers, mobile devices, servers).

Medical records can comprise various sections or portions in various arrangements or layouts. For example, sections may have various degrees of structure. Highly structured sections of a medical record may include, for example, tables with headers and values arranged in rows and columns. Less structured sections may include key-value pairs or typed or written text (e.g., narrative text and/or notes). Templates can comprise layouts or arrangements of various sections with different characteristics. It would be advantageous to detect templates to determine respective sections, which may facilitate identification of appropriate extractors for extraction of medical data.

Regardless of the process or manner by which a medical record is generated, the disclosed technology can classify medical records to respective templates based on characteristics of the medical record to facilitate medical data extraction. For example, the disclosed technology can determine sections or portions of a medical record that use key-value pairs, which allows for improved extraction of the medical data in the medical record. Additionally, the disclosed technology can identify sections or portions of a medical record that include a table to facilitate extraction of medical data from the table. The disclosed technology can further identify portions of a medical record that include narrative text (e.g., notes) to facilitate extraction of medical data comprising narrative text. These and other sections or portions of a medical record can be identified as parts of a template. By detecting the template according to which a medical record is prepared, the disclosed technology can extract medical data from the medical record with improved accuracy and efficiency. For example, appropriate extractors can be selected for extracting medical data from different sections or portions of the medical record, such as using a table parser for detected tables or one or more language models (e.g., named entity recognition (NER), free-text NLP) for detected narrative text. In other words, in some applications, template detection may allow application of specific models or extractors on different portions of the medical record, which may improve extraction compared to applying a single model or extractor to the entire medical record.

Various embodiments can perform garbage detection to evaluate results of template detection and/or medical data extraction. For example, a trained model can be applied to detect a template for a received medical record, and medical data can be extracted from the received medical record based on the detected template. The extracted medical data can be evaluated (e.g., based on known medical data) to estimate accuracy of the extracted medical data. Various actions can be performed based on the garbage detection, such as discarding the extracted medical data when the estimated accuracy does not exceed a threshold value. Garbage detection can additionally or alternatively include discarding medical records and/or associated data when no template can be detected for the medical records.

Advantages of the disclosed technology include, without limitation, increased efficiency and reduced burden related to review (e.g., manual review) of medical records, improved support for decision making or diagnoses, improvements to automated billing processes, and so forth. For example, the disclosed technology improves over existing systems, which may require manual review of medical records for manual entry of medical data into a patient record. The disclosed technology also improves over existing systems that may have limited functionality to automatically extract data, but are unable to accurately and efficiently extract medical data from medical records that do not use a known template. For example, existing systems may be able to perform extraction of medical data only when the template type is already known to the systems and only when medical records rigidly conform to the known template type. The disclosed technology also improves accuracy and quality of extracted medical data, such as by identifying medical records that do not follow a template or medical data that does not exceed a threshold accuracy to be discarded or corrected.

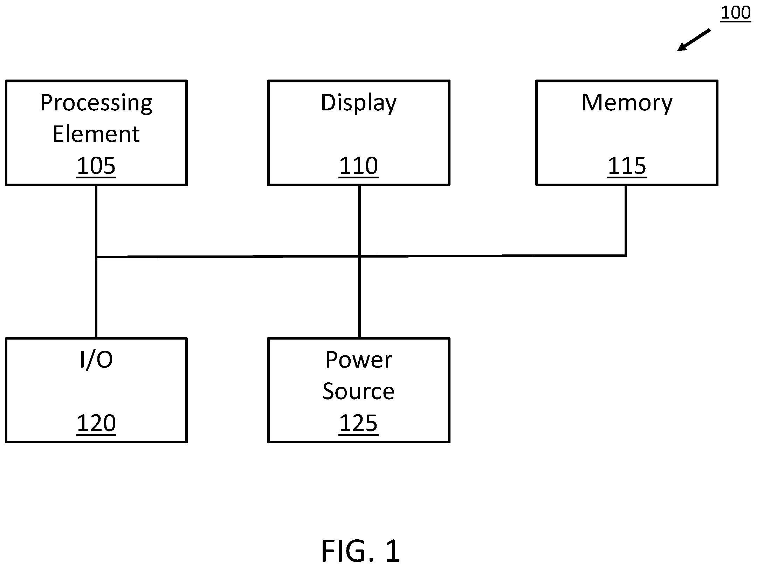

Fig. 1 is a block diagram illustrating a computing device 100 implementing a system for medical data extraction, in some implementations. For example, the computing device 100 can be one or more servers and/or user devices.

The computing device 100 includes one or more processing elements 105, displays 110, memory 115, an input/output interface 120, and/or power sources 125, each of which may be in communication either directly or indirectly.

The processing element 105 can be any type of electronic device and/or processor capable of processing, receiving, and/or transmitting instructions. For example, the processing element 105 can be a microprocessor or microcontroller. Additionally, it should be noted that select components of the system may be controlled by a first processor and other components may be controlled by a second processor, where the first and second processors may or may not be in communication with each other. The device 100 may use one or more processing elements 105 and/or may utilize processing elements included in other components. For example, a first device 100 can be a user device, while a second device 100 can be a server, and the first device 100 can use a processing element 105 of the second device.

The display 110 provides visual output to a user and optionally may receive user input (e.g., through a touch screen interface). The display 110 may be substantially any type of electronic display, including a liquid crystal display, organic liquid crystal display, and so on. The type and arrangement of the display depends on the desired visual information to be transmitted (e.g., can be incorporated into a wearable item such as glasses, or may be a television or large display, or a screen on a mobile device).

The memory 115 stores data used by the device 100 to store instructions for the processing element 105, and the memory 115 stores data for the system, such as extracted medical data, template information, template and/or data repositories, one or more trained models, and so forth. The memory 115 may be, for example, magneto-optical storage, read only memory, random access memory, erasable programmable memory, flash memory, or a combination of one or more types of memory components. The memory 115 can include, for example, one or more non-transitory computer-readable media carrying instructions configured to cause the processing element 105 and/or the device 100 or other components of the system to perform operations described herein.

The I/O interface 120 provides communication to and from the various components within the device 100. The I/O interface 120 can include one or more input buttons, switches, or other input devices (e.g., keyboard, control pad, touch pad, mouse). The I/O interface 120 may further include a communication interface, such as WiFi, Ethernet, or the like, as well as other communication components, such as universal serial bus (USB) cables, or the like. In some implementations, the I/O interface 120 can be configured to receive voice inputs and/or gesture inputs. The I/O interface 120 may receive inputs from users or other systems. For example, a user may provide inputs via a keyboard or electronic medical data may be provided via Ethernet. The I/O interface may provide inputs to the appropriate components. For example, user inputs may be provided to the processing element 105 and electronic medical data may be provided to the processing element 105 and/or memory 115.

The power source 125 provides power to the various computing resources and/or devices. The system may include one or more power sources, and the types of power source may vary depending on the component receiving power. The power source 125 may include one or more batteries, wall outlet, cable cords (e.g., USB cord), or the like.

Components of the device 100 are illustrated only as examples, and illustrated components can be removed from and/or added to the device 100 without deviating from the teachings of the present disclosure.

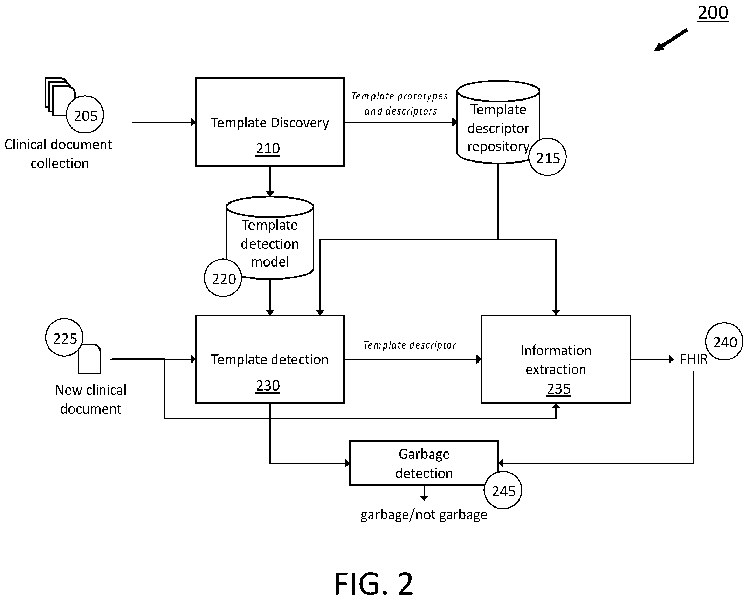

Fig. 2 is a block diagram illustrating a workflow 200 for medical data extraction in accordance with principles of the present disclosure. As used herein, a "module" can refer to one or more computer executable instructions that may be part of a larger set of computer executable instructions (e.g., software program). The instructions may be executed by one or more processors, such as processing element 105. The instructions and data analyzed by the processor based on the instructions may be stored in one or more memories, such as memory 115.

A clinical document collection is received comprising a plurality of medical records 205 (e.g., hundreds or thousands of medical records 205). The medical records 205 can be stored in the memory 115 and/or received via the I/O interface 120. The medical records 205 can be paper records, physical records (e.g., medical images), and/or electronic records, and the medical records can have respective templates that may not be previously known to the disclosed system. In some implementations, the medical records 205 are received in or converted to a particular digital format, such as portal document format (PDF) with optical character recognition (OCR). Additionally or alternatively, computer vision or similar technology can be used to identify characteristics of the medical records 205. In some embodiments, the medical records 205 comprise images that are converted to text. In some implementations, the medical records 205 are received as or converted to raw text.

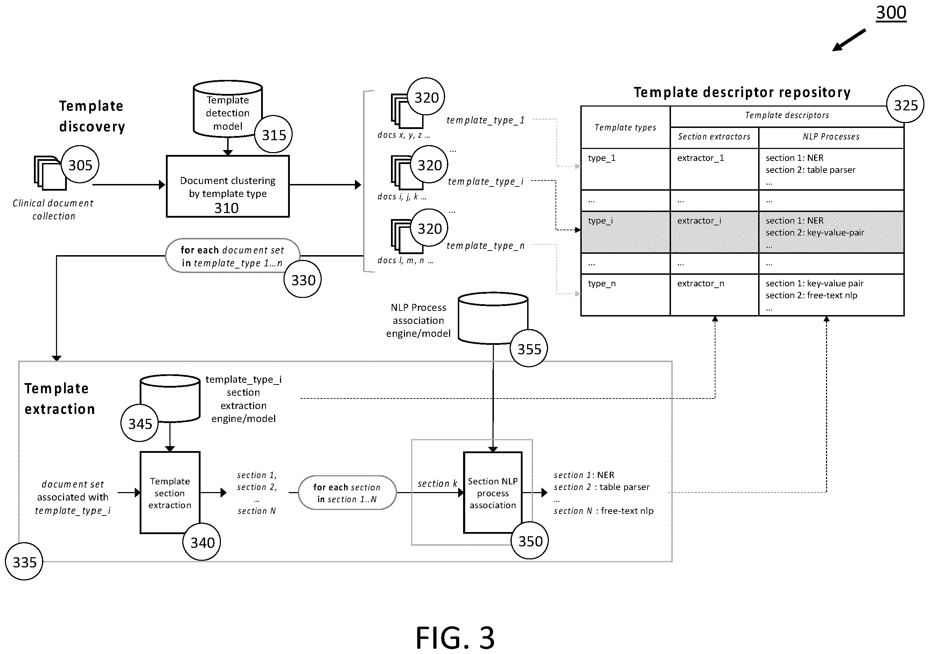

A template discovery module 210 processes the medical records 205 to discover template formats of the templates, as further discussed with reference to the workflow 300 of Fig. 3. The template discovery module 210 can comprise instructions stored in the memory 115 and executed using the processing element 105. Discovered templates are abstracted into template prototypes, and one or more template descriptors are determined based on characteristics of the medical records 205. A template prototype can be a representation of a template, which can be used to performing clustering of medical records 205 into respective document sets. A template descriptor can include an overall layout, fixed parts, and/or variable parts of the template. Template descriptors can describe sections and/or subsections of a medical record. A template descriptor can include operations to extract data from different template sections and a mapping between each section and an extractor (e.g., a language model) to extract data from that section. Template descriptors and template prototypes are stored in a template descriptor repository 215. The template descriptor repository 215 can comprise a database and/or can be stored in a memory (e.g., memory 115). Based on the template formats discovered by the template discovery module 210 and/or the template descriptors and template prototypes stored in the template descriptor repository 215, one or more template detection models 220 are provided and/or trained to perform template detection, as further discussed with reference to the workflow 300 of Fig. 3. A template detection model 220 can comprise a machine learning model, which may be implemented by computer readable instructions in some embodiments. In various embodiments, the machine learning model can use an unsupervised approach, such as a clustering algorithm. In these and other implementations, the template detection model 220 can include classification algorithms, such as text or image classification models, which can be applied, for example, to annotations of a template or medical record.

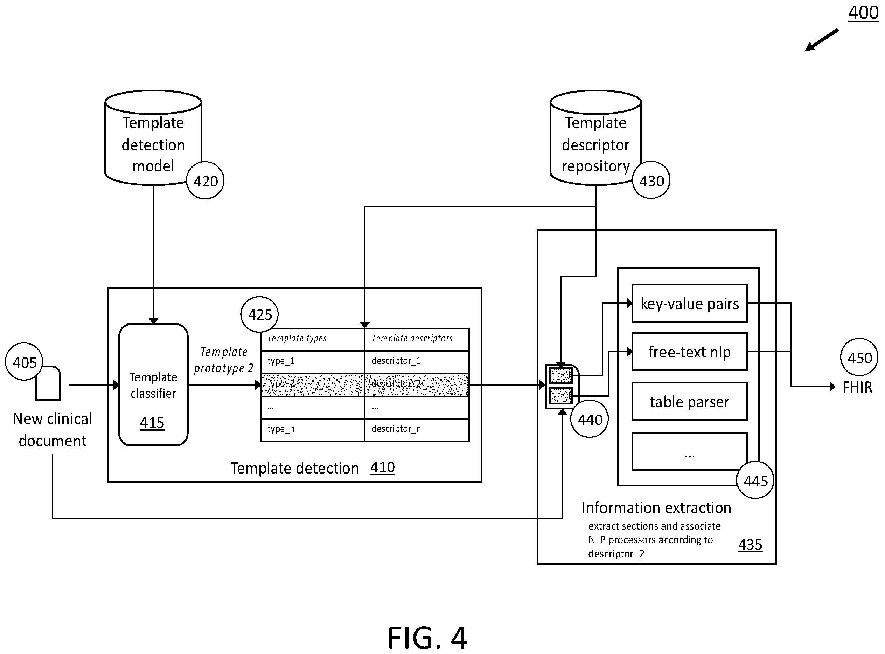

After a template detection model 220 has been trained, it can be applied to perform template detection, including classifying a received medical record 225 (e.g., a new clinical document) to an appropriate template. A template detection module 230 accesses the medical record 225. The template detection module 230 can comprise instructions stored in the memory 115 and executed by the processing element 105, and the medical record 225 can be received via the I/O interface 120. The template detection module 230 applies the template detection model 220 to detect a template for the medical record 225, as further discussed with reference to Figs. 3 and 4. For example, the template detection model 220 identifies the overall layout and any fixed and/or variable parts of the template to determine that the medical record 225 has a particular format (e.g., based on one or more template descriptors and/or template prototypes). Detecting the template by the template detection module 230 can include using the template detection model 220 to determine one or more confidence scores indicative of a likelihood that the medical record 225 has a respective template. A template can be detected, for example, based on a confidence score exceeding a threshold value and/or based on a highest confidence score of a set of confidence scores (e.g., different scores for different templates).

After the template for the medical record 225 has been detected, the medical record 225 and the detected template are accessed by an information extraction module 235, which extracts medical data from the medical record 225 based on the detected template to generate output data 240 (e.g., extracted medical data). For example, the information extraction module 235 can select an appropriate language model (e.g., NLP model) and/or other extractor to extract medical data from the medical record 225, and the information extraction module 235 can apply the selected model to the medical record 225. The information extraction module 235 can comprise instructions stored in the memory 115 and executed by the processing element 105. In various embodiments, the information extraction module 235 transforms and/or formats the output data 240 according to one or more standards, such as to generate FHIR data, or a desired non-standard format. The information extraction module 235 can include a set of language models (e.g., NLP models) configured to process one or more fields of a detected template. The information extraction module 235 can identify one or more extractors to extract the medical data from the medical record 225 based on the detected template, such as NLP models or table parsers. In some embodiments, different NLP models and/or extractors may be used for different templates and/or different fields of the detected template. The output data 240 can be provided to the display 110 and/or via the I/O interface 120.

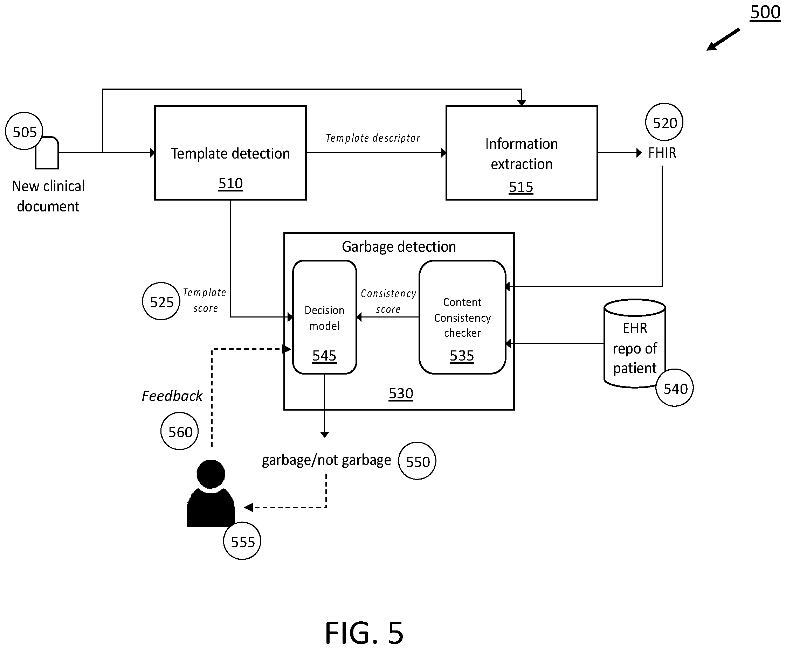

In various embodiments, a garbage detection module 245 performs garbage detection to evaluate a medical record 225 and/or output data 240, as further discussed with reference to the workflow 500 of Fig. 5. The garbage detection module 245 can comprise instructions stored in the memory 115 and executed by the processing element 105. In some examples, the garbage detection module 245 determines that no template can be detected for a medical record 225 (e.g., based on comparing one or more confidence scores determined by the template detection module 230 to a threshold value). Additionally or alternatively, the garbage detection module 245 evaluates accuracy of output data 240. For example, at least a portion of the output data 240 can be compared to known subject data to determine accuracy. In a specific example, output data 240 associated with a patient name in a detected template can be compared to a known patient name from an existing medical record. If the extracted patient name matches the known patient name beyond a threshold accuracy, then this indicates a likelihood that the output data 240 is accurate. On the other hand, if the extracted patient name does not match the known patient name beyond a threshold accuracy, then this indicates a likelihood that the output data 240 is inaccurate. In some implementations, output data 240 and/or medical records 225 are discarded based on garbage detection, such as when no template can be detected or when the output data 240 does not exceed a threshold accuracy. In these and other embodiments, one or more portions of the workflow 200 can be repeated for the output data 240 and/or medical records 250 to determine whether accuracy can be improved. Other actions can additionally or alternatively be taken, such as notifying a user that garbage has been detected and/or prompting a user to provide an input (e.g., confirmation that output data 240 is inaccurate or the like). User notifications or prompts can be provided via the display 110.

As used herein, a "model" can refer to a construct that is trained using training data to make predictions or provide probabilities for new data items, whether or not the new data items were included in the training data. For example, training data for supervised learning can include items with various parameters and an assigned classification. A new data item can have parameters that a model can use to assign a classification to the new data item. As another example, a model can be a probability distribution resulting from the analysis of training data, such as a likelihood of an n-gram occurring in a given language based on an analysis of a large corpus from that language. Examples of models and/or associated techniques include, without limitation: neural networks, support vector machines, decision trees, Parzen windows, Bayes, clustering, reinforcement learning, probability distributions, decision trees, decision tree forests, and others. Models can be configured for various situations, data types, sources, and output formats. A model trained or provided by the disclosed system can include a neural network with multiple input nodes that receive training datasets. The input nodes can correspond to functions that receive the input and produce results. These results can be provided to one or more levels of intermediate nodes that each produce further results based on a combination of lower-level node results. A weighting factor can be applied to the output of each node before the result is passed to the next layer node. At a final layer, ("the output layer,") one or more nodes can produce a value classifying the input that, once the model is trained, can be used to evaluate medical records (e.g., to detect templates). In some implementations, such neural networks, known as deep neural networks, can have multiple layers of intermediate nodes with different configurations, can be a combination of models that receive different parts of the input and/or input from other parts of the deep neural network, or are convolutions-partially using output from previous iterations of applying the model as further input to produce results for the current input.

A model can be trained with supervised learning (e.g., self-supervised). Testing data can then be provided to the model to assess accuracy. Testing data can be, for example, a portion of the entire dataset (e.g., 10%) held back to use for evaluation of the model. Output from the model can be compared to the desired or expected output for the training data and, based on the comparison, the model can be modified, such as by changing weights between nodes of the neural network and/or parameters of the functions used at each node in the neural network (e.g., applying a loss function). Based on the results of the model evaluation, and after applying the described modifications, the model can then be retrained to evaluate new data.

Fig. 3 is a block diagram illustrating a workflow 300 for template discovery and extraction in accordance with principles of the present disclosure. The workflow 300 can be used, for example, to train a template detection model 315 and apply the trained model to perform medical data extraction. In some embodiments, template detection model 315 may include template detection model 220.

A clinical document collection is received comprising a plurality of medical records 305 (e.g., 205 of Fig. 2), such as hundreds or thousands of medical records. The medical records 305 may follow various templates, which may not be previously known to the disclosed system. At an operation 310, a template detection model 315 is applied to perform clustering of the medical records 305 based on detected template types. The template detection model 315 identifies document sets 320 of the medical records 305, each document set 320 having a respective template type. For example, the document sets 320 can be identified based on similarity scores generated for the medical records 305, such that medical records 305 having similar parts or sections (e.g., tables, key-value pairs, narrative text), similar layouts or arrangements, and/or other characteristics are clustered to generate the document sets 320, each document set 320 associated with a template type.

The document sets 320 are used to generate and/or populate a template descriptor repository 325 (e.g., 215 of Fig. 2). The template descriptor repository 325 determines and/or stores characteristics of template types associated with the respective document sets. The template characteristics include a template type (e.g., type_1 to type_n) and respective template descriptors, extractors, data types, and/or processes or models for extracting medical data of portions of medical records based on the template types (e.g., named entity recognition (NER), table parser, key-value pairs, free-text NLP).

At an operation 330, each document set 320 is provided to perform a template extraction operation 335. In some embodiments, extraction operation 335 may be included in or performed by extraction module 235. For each document set 320, the template extraction operation 335 includes performing a template section extraction operation 340 using a template section extraction model 345 (e.g., an extractor) to extract (e.g., detect or identify) sections (e.g., portions) of a template of the document set 320. In other words, the template section extraction model 345 detects sections or portions of a template for medical records in the document set 320. For the detected sections, the template extraction operation 335 further performs a section NLP process association operation 350 using a NLP process association model 355. The section NLP processor association operation 350 associates each section of a medical record in a document set 320 with template descriptors, extractors, and/or processors or models for extracting medical data (e.g., NER, table parser, free-text NLP). Different sections (e.g., section 1, section 2, section N) of the medical record may be associated with different descriptors, extractors, processors, and/or models. As shown in the example in Fig. 3, section 1 is associated with a NER and section 2 is associated with a table parser.

The results of the template extraction operation 335 can be evaluated based on the template descriptor repository 325 and/or using ground truth data, and the evaluation can be used to train one or more of the template detection model 315, the template section extraction model 345, or the NLP process association model 355. In some implementations, a single model comprises the template detection model 315, the template section extraction model 345, and the NLP process association model 355, and the workflow 300 is used to train the single model. In these and other implementations, a training dataset can be generated or provided using the medical records 305, which can include annotations and/or known templates, template types, sections or portions, extractors, language models or processes, or the like, which can be used as ground truth data for training. The results of the template extraction operation 335 can be evaluated using the training dataset, and one or more loss functions can be generated to evaluate accuracy of the template detection model 315, the template section extraction model 345, or the NLP process association model 355. When accuracy of one or more of these models does not exceed a threshold accuracy, at least a portion of the workflow 300 can be repeated to continue training and/or to retrain the model using adjusted weights and/or training data. When an acceptable level of accuracy is achieved, the trained model can be applied to extract data from new (e.g., previously unseen) medical records based on template detection.

Fig. 4 is a block diagram illustrating a workflow 400 for template detection in accordance with principles of the present disclosure. The workflow 400 applies a template detection model 420 (e.g., 220 of Fig. 2 or 315 of Fig. 3) to extract medical data from a medical record 405.

A new (e.g., previously unseen) clinical document comprising a medical record 405 is accessed by a template detection module 410 (e.g., 230 of Fig. 2). A template classifier 415 of the template detection module 410 applies a template detection model 420 to classify the medical record 405 to an appropriate template. Classifying the medical record 405 can include generating confidence scores based on the medical record 405 for a set of template types, such as template type_1 to template type_n, wherein each template type is associated with corresponding template descriptors, as illustrated in the table 425 of the template detection module 410. Template information, including corresponding template descriptors, are provided using a template descriptor repository 430 (e.g., 205 of Fig. 2 or 325 of Fig. 3). The confidence scores indicate a likelihood that the medical record 405 corresponds to a respective template based on various characteristics of the medical record, such as types and arrangements or layouts of medical data in the medical record 405 (e.g., tables, key-value pairs, narrative text).

An information extraction module 435 (e.g., 235, of Fig. 2 or 335 of Fig. 3) receives an input 440 that includes the medical record 405 and the template for the medical record 405 detected by the template detection module 410. Additionally, the input 440 includes descriptors, extractors and/or parts/sections associated with the template, which can be received from the template descriptor repository 430 or the template detection module 410. Based on the template and the descriptors, the information extraction module 435 selects and applies a set of extractors 445, which can comprise processes or models configured to extract medical data from the medical record 405. The information extraction module 435 formats the extracted data in a standard format, such as a format complying with the FHIR standard, and provides the formatted data as output data 450.

In an example implementation, the medical record 405 uses a template that includes a first section having a set of key-value pairs, a second section having a table with headers and values arranged in rows and columns, and a third section having narrative text. The template detection module 410 evaluates the layout and sections of the medical record 405 and determines template descriptors and/or other template characteristics based on the evaluation. The template detection module 410 generates confidence scores indicating a likelihood that the medical record 405 uses respective templates of a set of templates, based on template types and corresponding descriptors stored in the template descriptor repository 430. Based on the confidence scores (e.g., by selecting a highest confidence score), the template detection module 410 classifies the medical record 405 to a particular template of the set of templates. The particular template matches the layout and the sections of the medical record 405 (e.g., beyond a threshold confidence). The particular template is associated with a set of descriptors, extractors, and/or processes or models for data extraction in the template descriptor repository 430. The information extraction module 435 selects appropriate extractors for each of the first section, the second section, and the third section based on the detected template for the medical record 405. For example, based on the detected template, the information extraction module 435 determines that a key-value pair tool is needed to extract medical data from the first section (e.g., keys and associated values), a table parser is needed to extract medical data from the second section (e.g., headers and values in the table), and a free-text NLP is needed to extracted medical data from the third section (e.g., narrative text). The information extraction module 435 applies the extractors to the medical record 405 to extract the medical data from the respective sections, and the information extraction module 435 formats the extracted data using a standard format to provide the output data 450.

Fig. 5 is a block diagram illustrating a workflow 500 for garbage detection in accordance with principles of the present disclosure.

A new clinical document comprising a medical record 505 (e.g., 405 of Fig. 4) is accessed. A template detection module 510 (e.g., 410 of Fig. 4) performs template detection to detect a template of the medical record 505, and an information extraction module 515 (e.g., 435 of Fig. 4) extracts medical data from the medical record 505. Template detection and medical data extraction are performed as described with reference to the workflow 400 of Fig. 4.

The information extraction module 515 provides output data 520 comprising extracted medical data in a standard format, as described with reference to Fig. 4, and the template detection module 510 provides a detected template and a template score 525, which is a confidence score indicating a likelihood that the medical record 505 uses the detected template. The template score 525 is generated by a template detection model (e.g., 420 of Fig. 4). The output data 520 and the template score 525 are received by a garbage detection module 530 (e.g., 245 of Fig. 2) to perform garbage detection. As used herein, "garbage detection" can refer to one or more operations or processes to evaluate a detected template and/or extracted data (e.g., unformatted extracted data and/or output data 520) associated with a medical record 505 and determine accuracy of the same. When accuracy of the detected template and/or extracted data does not exceed a threshold accuracy or confidence (e.g., 70%, 80%, 90%, 100%), various actions can be taken, such as excluding or discarding extracted data and/or a template determination, notifying a user, prompting a user for a confirmation, or the like.

A consistency checker 535 of the garbage detection module 530 receives at least a portion of the output data 520 and/or extracted data used to generate the output data 520, which includes medical data extracted from the medical record 505. The consistency checker further receives corresponding data from a repository 540, which comprises known data of a subject, such as an electronic health record (EHR) of a patient. The repository 540 can be a database, and the repository 540 can be stored in a memory (e.g., 115 of Fig. 1) and/or accessed from an external source (e.g., via 120 of Fig. 1). In an example implementation, the information extraction module 515 determines that a key-value pair is present in the medical record 505 based on a template detected by the template detection module 510, wherein the key-value pair corresponds to a name field and a subject name in the name field, and the information extraction module 515 identifies and applies an appropriate extractor (e.g., a NLP model) to extract the name value from the medical record 505. The consistency checker 535 receives the extracted name value and a known name of a subject from the repository and compares the extracted name to the known name. In some implementations, the consistency checker 535 performs a simple string comparison. In these and other implementations, the consistency checker 535 generates one or more similarity scores to evaluate similarity of the output data 520 to known data from the repository 540. The consistency checker 535 provides a consistency score to a decision model 545, which indicates a likelihood that the output data 520 is accurate. In the example discussed above, when the value extracted from the name field matches the known subject name, the consistency checker 535 outputs a relatively high consistency score, indicating a high likelihood that the output data 520 is accurate. A low consistency score indicates a low likelihood that the output data 520 is accurate, such as when at least a portion of the output data 520 does not match data in the repository 540.

A low consistency score may be caused by one or more factors. For example, a type of field of the medical record 505 may have been misidentified (e.g., a narrative text field identified as a key-value pair field). In another example, a section of the medical record 505 may have been misidentified (e.g., a narrative text field for clinical results identified as a narrative text field for patient description of symptoms). In another example, a template of the medical record 505 has been incorrectly detected, and medical data has been incorrectly extracted or interpreted based on the incorrect detection. Other factors may cause low consistency scores in other cases.

The decision model 545 further receives the template score 525 from the template detection module 510, and the decision model 545 generates an output 550 based on the template score 525, the consistency score provided by the consistency checker 535, or both. For example, the output 550 can indicate whether the detected template and/or the output data 520 is accurate. The output 550 can be used to automatically take one or more actions, such as discarding a detection of a template, discarding the output data 520, repeating at least a portion of the workflow 500, and so forth.

In some implementations, the output 550 is provided to a user 555 (e.g., via a user interface and/or on a display, such as display 110), and the user 555 is prompted to provide a confirmation of the output 550. For example, when the output data 520 is determined to be inaccurate (e.g., when accuracy does not exceed a threshold level), a notification can be generated to notify the user 555 that the output data 520 is determined to be inaccurate, and the user 555 can provide feedback 560 (e.g., to the decision model 545) to agree or disagree with the determination that the output data 520 is inaccurate. Additionally or alternatively, the user can provide feedback 560 regarding whether a template for the medical record 505 was accurately detected. For example, a notification can be generated to notify the user 555 of a detected template for the medical record 505 or, alternatively, that no template could be identified for the medical record. In these and other implementations, the feedback 560 can include a correct template for the medical record 505 or an indication that the medical record 505 does not correspond to a template. In some embodiments, the user 555 may correct the output data 520 (e.g., provide correct subject name). User feedback can be used to train, retrain, and/or modify the disclosed system. For example, output data 520 that is indicated the user 555 to be accurate can be stored in the repository 540 to be used by the consistency checker 535 for comparison to other extracted medical data from other medical records 505. Additionally or alternatively, feedback 560 related to accuracy of template detection can be used to train, retrain, or modify a template detection model.

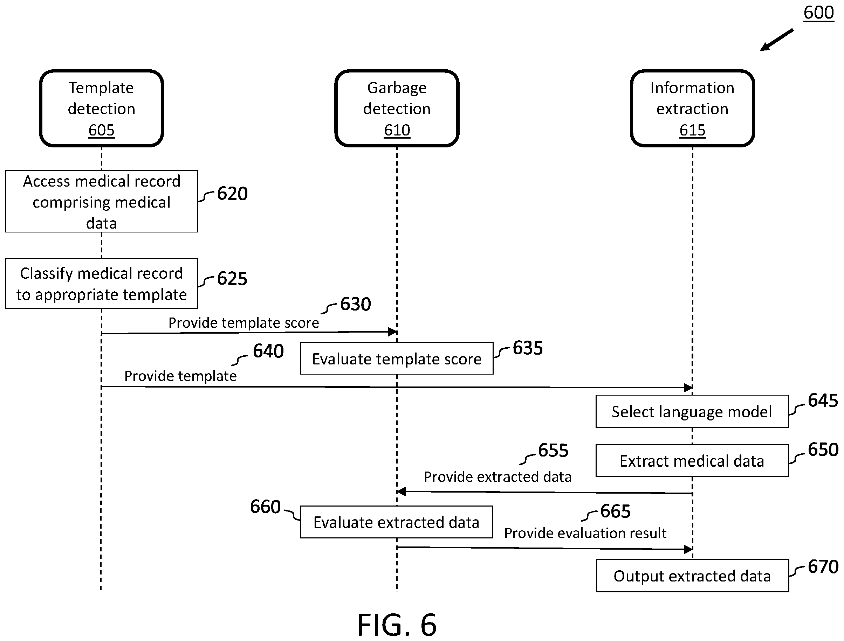

Fig. 6 is a sequence diagram illustrating a process (or computer implemented method) 600 for templatizing medical records data in accordance with principles of the present disclosure. The process 600 is performed using a template detection module 605 (e.g., 230 of Fig. 2, 510 of Fig. 5), a garbage detection module 610 (e.g., 245 of Fig. 2, 530 of Fig. 5), and an information extraction module 615 (e.g., 235 of Fig. 2, 515 of Fig. 5). In some embodiments, the process 600 may be implemented by a computing device, such as computing device 100 of Fig. 1.

At operation 620, the template detection module 605 accesses a medical record comprising medical data. The medical record has a template comprising an arrangement or layout of the medical data having one or more sections that are structured, semi-structured, and/or unstructured. For example, the template can comprise one or more tables or other data structures, narrative text, and/or key-value pairs arranged in a physical layout.

At operation 625, the template detection module 605 applies a trained model (e.g., a machine learning model, which can be a template detection model) to classify the accessed medical record according to the template. For example, a set of template scores can be determined and used to identify a template based on the template scores, such as based on a template score threshold and/or a highest template score of a set of template scores. Classifying the medical record can include or be based on one or more descriptors characterizing the template and/or the medical record, such as descriptors associated with the layout, the sections, and/or other characteristics of the template or the medical record. The template detection module 605 analyzes the medical record to determine a template of a set of templates that most closely matches the characteristics of the medical record.

At operation 630, the template detection module 605 provides a template score to the garbage detection module 610. For example, the template detection module 605 can provide a highest template score of a set of template scores determined at operation 625 (e.g., for a detected template of a set of templates).

At operation 635, the garbage detection module 610 evaluates the template score received at operation 630. In some implementations, the evaluation of the template score can indicate accuracy of the template score. For example, when the template score does not exceed a threshold value, the garbage detection module 610 determines that no template can be identified. When no template can be identified, one or more actions can be taken, such as to pause or terminate the process 600, discard a medical record and/or a template determination, repeat at least a portion of the process 600, and/or prompt a user for feedback (e.g., confirmation that no template can be detected or an indication of a correct template).

At operation 640, a determined template (as determined at operation 625) is provided to the information extraction module 615.

At operation 645, the information extraction module 615 selects a language model and/or other extractor to extract the medical data from the medical record based on the classification of the medical record (e.g., based on a detected template). In some implementations, different models are selected to extract data from different portions or sections of the medical record.

At operation 650, the information extraction module 615 extracts medical data from the medical record using the model selected at operation 645. For example, the information extraction module 615 can identify one or more extractors for extracting the medical data from the medical record based on extractors associated with templates in a template descriptor repository (e.g., 215 of Fig. 2, 325 of Fig. 3, 430 of Fig. 4), and the information extraction module 615 applies the identified extractors to respective sections of the medical record based on the template to extract the medical data. The extractors can be one or more NLP processors or models.

At operation 655, the information extraction module 615 provides the medical data extracted at operation 645 to the garbage detection module 610.

At operation 660, the garbage detection module 610 evaluates the extracted medical data received at operation 655. For example, the garbage detection module 610 can compare the extracted medical data to known data of a subject (e.g., from repository 540 of FIG. 5). A result of the evaluation at operation 660 can comprise an indication of accuracy of the extracted medical data, such as an indication of whether the extracted data matches the known data of the subject.

At operation 665, the results of the evaluation performed at operation 660 are provided to the information extraction module 615.

At operation 670, the information extraction module 615 outputs the extracted data. In some implementations, the information extraction module 615 outputs the extracted data only when the evaluation performed at operation 635 and/or operation 655 indicate accuracy of the extracted data beyond a threshold level. In some examples, output data is generated using the extracted data and according to a predetermined standard, such as the FHIR standard.

In some implementations, the process 600 includes training a model used by the template detection module 605. In some implementations, the process 600 includes evaluating accuracy of the trained model and retraining the model when accuracy of the trained model does not exceed a threshold accuracy.

More or fewer operations can be included in the process 600 without deviating from the teachings of the present disclosure, and one or operations can be repeated. In some implementations, one or more operations of the process 600 are performed in parallel.

The systems and related methods disclosed herein may provide for more accurate and efficient extracting of medical data from medical records compared to existing systems and methods for medical data extraction from medical records. The systems and methods disclosed herein may further allow for conversion of medical record data into different formats (e.g., FHIR standard format). The detection of templates of medical records may allow for different extraction techniques to be applied to different portions of medical records in some embodiments. This may allow for more efficient and/or accurate extraction of medical data from the medical record. In some applications, the improved accuracy may reduce the amount of time required for manual correction of output data generated from the extracted medical data. In some applications, the systems and related methods may reduce missing or incorrect medical data in output data, which may lead to improved medical care by providers relying on said output data.

Embodiments of the method may be executed using software, which comprises instructions for causing a processor system to perform the method disclosed herein. Software may only include those steps taken by a particular sub-entity of the system. The software may be stored in a suitable storage medium, such as a hard disk, a floppy, a memory, an optical disc, etc. The software may be sent as a signal along a wire, or wireless, or using a data network, e.g., the Internet. The software may be made available for download and/or for remote usage on a server or the cloud. Embodiments of the method may be executed using a bitstream arranged to configure programmable logic, e.g., a field-programmable gate array (FPGA), to perform the method.

In various examples where components, systems and/or methods are implemented using a programmable device, such as a computer-based system or programmable logic, it should be appreciated that the above-described systems and methods can be implemented using any of various known or later developed programming languages, such as "Python", "C", "C++", "FORTRAN", "Pascal", "VHDL" and the like. Accordingly, various storage media, such as magnetic computer disks, optical disks, electronic memories and the like, can be prepared that can contain information that can direct a device, such as a computer, to implement the above-described systems and/or methods. Once an appropriate device has access to the information and programs contained on the storage media, the storage media can provide the information and programs to the device, thus enabling the device to perform functions of the systems and/or methods described herein. For example, if a computer disk containing appropriate materials, such as a source file, an object file, an executable file or the like, were provided to a computer, the computer could receive the information, appropriately configure itself and perform the functions of the various systems and methods outlined in the diagrams and flowcharts above to implement the various functions. That is, the computer could receive various portions of information from the disk relating to different elements of the above-described systems and/or methods, implement the individual systems and/or methods and coordinate the functions of the individual systems and/or methods described above.

A computer readable medium according to the present invention may comprise a writable part 1010. Computer readable medium may is implemented in the form of an optically readable medium, an electronic memory (e.g. a memory card) or any suitable computer readable medium, such as a hard disk, solid state memory, flash memory, etc., and may be non-recordable or recordable. Computer readable medium may store data wherein the data may indicate instructions, which when executed by a processor system, cause a processor system to perform an embodiment of a method disclosed herein. The computer program may be embodied on the computer readable medium as physical marks or by magnetization of the computer readable medium. However, any other suitable embodiment is conceivable as well. The computer program comprises instructions for causing a processor system to perform an embodiment of said method.

In view of this disclosure it is noted that the various methods and devices described herein can be implemented in hardware, software, and/or firmware. Further, the various methods and parameters are included by way of example only and not in any limiting sense. In view of this disclosure, those of ordinary skill in the art can implement the present teachings in determining their own techniques and needed equipment to affect these techniques, while remaining within the scope of the invention. The functionality of one or more of the processors described herein may be incorporated into a fewer number or a single processing unit (e.g., a CPU) and may be implemented using application specific integrated circuits (ASICs) or general-purpose processing circuits which are programmed responsive to executable instructions to perform the functions described herein.

Of course, it is to be appreciated that any one of the examples or processes described herein may be combined with one or more other examples and/or processes or be separated and/or performed amongst separate devices or device portions in accordance with the present systems, devices and methods.

Finally, the above-discussion is intended to be merely illustrative of the present systems and methods and should not be construed as limiting the appended claims to any particular example or group of examples. Thus, while the present system has been described in particular detail with reference to exemplary examples, it should also be appreciated that numerous modifications and alternative examples may be devised by those having ordinary skill in the art without departing from the broader and intended spirit and scope of the present systems and methods as set forth in the claims that follow. Accordingly, the specification and drawings are to be regarded in an illustrative manner and are not intended to limit the scope of the appended claims.

## Claims

1. A computer-implemented method (600) for automatically templatizing medical records data, the method comprising:
accessing a medical record comprising medical data (620);
classifying, by a trained model, the accessed medical record to an appropriate template comprising one or more of a layout, a key-value pair, or a data structure corresponding to the accessed medical record (625);
selecting a language model based on the classified template;
extracting the medical data from the accessed medical record by applying the selected language model to the accessed medical record in accordance with the classified template (645); and
generating, using the extracted medical data, output data according to a predetermined standard (665).

2. The computer-implemented method of claim 1, wherein the trained model comprises a classifier model that is trained by:
accessing a plurality of medical records (205) comprising a plurality of respective medical data, wherein the plurality of medical records have respective templates;
generating a training dataset using the plurality of medical records, wherein generating the training dataset includes identifying at least one data structure in the plurality of medical records; and
training, using the generated training dataset, the classifier model (220) to detect templates of received medical records to perform medical data extraction.

3. The computer-implemented method of claim 2, wherein training the classifier model further comprises:
evaluating the trained classifier model using a testing dataset to determine an accuracy of the trained classifier model, wherein the testing dataset comprises a portion of the generated training dataset; and
retraining the trained classifier model when the accuracy of the trained classifier model does not exceed a threshold accuracy, wherein retraining the classifier model includes adjusting a weight associated with the classifier model or training the classifier model at least a second time.

4. The computer-implemented method of claim 2, wherein training the classifier model includes generating a template descriptor repository (215) comprising a plurality of template types and template descriptors for the template types, wherein the template descriptors include section extractors associated with the template types, the section extractors comprising a parser, a natural language processing (NLP) model, or both.

5. The computer-implemented method of claim 1, wherein classifying the accessed medical record comprises generating a confidence score indicating a confidence that the medical record is associated with the template.

6. The computer-implemented method of claim 5, further comprising:
discarding the accessed medical record when the confidence score does not exceed a threshold.

7. The computer-implemented method of claim 1, wherein classifying the accessed medical record includes determining a set of template descriptors associated with the medical record, and wherein extracting the medical data includes identifying at least one extractor to extract the medical data based on the set of template descriptors.

8. A computing system (100) comprising:
at least one processor (105); and
at least one non-transitory memory (115) carrying instructions configured to cause the computing system to perform operations comprising:
accessing a medical record comprising medical data (620);
classifying, by a trained model, the accessed medical record to an appropriate template comprising one or more of a layout, a key-value pair, or a data structure corresponding to the accessed medical record (625);
selecting a language model based on the classified template;
extracting from the accessed medical data from the accessed medical record by applying the selected language model to the classified template (645); and
generating, using the extracted medical data, output data according to a predetermined standard (665).

9. The computing system of claim 8, wherein the trained model comprises a classifier model that is trained by:
accessing a plurality of medical records (205) comprising a plurality of respective medical data, wherein the plurality of medical records have respective templates;
generating a training dataset using the plurality of medical records, wherein generating the training dataset includes identifying at least one data structure in the plurality of medical records; and
training, using the generated training dataset, the classifier model (220) to detect templates of received medical records to perform medical data extraction.

10. The computing system of claim 9, wherein the operations further comprise:
evaluating the trained classifier model using a testing dataset to determine an accuracy of the trained classifier model, wherein the testing dataset comprises a portion of the generated training dataset; and
retraining the trained classifier model when the accuracy of the trained classifier model does not exceed a threshold accuracy, wherein retraining the classifier model includes adjusting a weight associated with the classifier model or training the classifier model at least a second time.

11. The computing system of claim 9, wherein training the classifier model includes generating a template descriptor repository (215) comprising a plurality of template types and template descriptors for the template types, wherein the template descriptors include section extractors associated with the template types, the section extractors comprising a parser, a natural language processing (NLP) model, or both.

12. The computing system of claim 8, wherein classifying the accessed medical record comprises generating a confidence score indicating a confidence that the medical record is associated with the template.

13. The computing system of claim 12, wherein the operations further comprise:
discarding the accessed medical record when the confidence score does not exceed a threshold.

14. The computing system of claim 8, wherein classifying the accessed medical record includes determining a set of template descriptors associated with the medical record, and wherein extracting the medical data includes identifying at least one extractor to extract the medical data based on the set of template descriptors.

15. A transitory or non-transitory computer readable-medium comprising data representing instructions, which when executed by a processor system, cause the processor system to perform the method according to any of claims 1-7.
